# EUROPEAN PATENT APPLICATION

(11) **EP 4 821 516 A1**
(43) Date of publication of application: **14.10.2026**
(21) Application number: 25738444.6
(22) Date of filing: 08.01.2025
(51) Int. Cl.: A61L 31/06, A61L 31/04, A61L 31/14, A61F 2/82, A61F 2/958

(54) **VASCULAR STENT, VASCULAR STENT SYSTEM, AND METHOD FOR PREPARING SAME**

(30) Priority: 12.01.2024 CN 202410052874
(71) Applicant: Shanghai MicroPort Medical (Group) Co., Ltd., Shanghai 201203 (CN)
(72) Inventor: LU, Jun, Shanghai 201203 (CN); WANG, Xueqin, Shanghai 201203 (CN); GU, Jialei, Shanghai 201203 (CN); ZHU, Jing, Shanghai 201203 (CN); ZHOU, Qi, Shanghai 201203 (CN)
(74) Representative: Marks & Clerk LLP
(86) International application number: PCT/CN2025/071217
(87) International publication number: WO 2025/148915

(57) **Abstract**

The present invention relates to the field of medical devices and provides a vascular stent, a vascular stent system and methods of fabrication thereof. The method for fabricating the vascular stent includes: preparing a first spinning solution by dissolving a biodegradable, water-insoluble polymer in a solvent and preparing a second spinning solution by dissolving a biodegradable, water-soluble polymer in a solvent; and introducing the first and second spinning solutions into electrospinning device and performing an electrospinning process, thereby forming the vascular stent. During delivery and expansion of the vascular stent, which is fabricated from the biodegradable, water-insoluble and water-soluble polymers by electrospinning, within the human body, biodegradable, water-soluble components fibers in the vascular stent will dissolve in bodily fluids, facilitating relative sliding and dislocation movements of the remainder of the fibers, and hence enhancing the stent's expansion properties and overexpansion capabilities.

## Description

### TECHNICAL FIELD

The present invention relates to the field of medical devices, and more particularly to a vascular stent, a vascular stent system and methods of fabrication thereof.

### BACKGROUND

Nowadays, biodegradable vascular stents have been widely used in the treatment of vascular diseases. In addition to coronary stenosis, vascular stents can be indeed used to treat conditions associated with all kinds of human blood vessels, from the thoracic and abdominal aortas, to intracranial and peripheral vessels.

For example, in the field of coronary treatment, biodegradable vascular stents have been recognized as a revolutionary innovation. Currently, most biodegradable stents are fabricated by laser engraving or multi-axis 3D printing techniques involving cutting a biodegradable polymer or metal tube into, or printing, a tubular structure consisting of struts and links. These stents are intended to provide scaffolding support for a while and end up with complete in vivo degradation.

Electrospinning is a fiber production method involving charging and stretching a liquid polymer droplet into a continuous jet under the action of an applied high-voltage electrostatic field. As solvent evaporation takes place at the same time, the jet solidifies into a micro/nanofiber assembly. Electrospinning vascular stents (referred to hereinafter as "vascular stents" for short) are three-dimensional (3D) stents made of micro/nanoscale fiber assemblies that mimic the extracellular matrix. Compared with stents with macroscopic openings, vascular stents are uniform 3D networks, which generate lower shear stress and do not induce localized turbulent flow. Parameters of related processes may be appropriately controlled to obtain vascular stents with desirable fiber diameters, pore sizes and porosities, which enable the stents to induce cell adhesion and proliferation and facilitate tissue regeneration, restoring vascular functionality.

During the use of a stent in an interventional procedure, it is crimped to a smaller diameter outside the patient's body, and once it is delivered to a target lesion site, a pressurization device is used to inflate a balloon, thereby expanding the stent and tightly pressing it against a vessel wall at the lesion site. Finally, the balloon is withdrawn.

Unlike traditional strut-based stents, vascular stents are 3D structures consisting of interwoven micro/nanofibers. However, due to a lack of deformability, little relative sliding and dislocation movements are allowed between fibers in the vascular stents when they are stressed during circumferential crimping and expansion. Moreover, when they are overly stretched during expansion, many fibers may be broken into pieces, leading to dramatic degradation in their mechanical properties. Therefore, the vascular stents disallows much overexpansion, and in severe cases, fracture may occur during expansion.

Therefore, there is a need for a novel vascular stent, vascular stent system and methods of fabrication thereof, which mitigate the problem of post-crimping-and-expansion degradation in scaffolding performance and mechanical properties associated with conventional vascular stents constructed as 3D structures from fibers.

### SUMMARY

It is just an object of the present invention to provide such a novel vascular stent, vascular stent system and methods of fabrication thereof, which mitigate the problem of post-crimping-and-expansion degradation in scaffolding performance and mechanical properties associated with conventional vascular stents constructed as 3D structures from fibers.

The present invention provides a method of fabricating a vascular stent, which includes:
preparing a first spinning solution by dissolving a biodegradable, water-insoluble polymer in a solvent and preparing a second spinning solution by dissolving a biodegradable, water-soluble polymer in a solvent; and
introducing the first and second spinning solutions into electrospinning device and performing an electrospinning process, thereby forming the vascular stent.

Optionally, the first and second spinning solutions may be simultaneously introduced into the electrospinning device, wherein coaxial fibers are produced and interwoven in the electrospinning process to form at least part of the vascular stent,
each of the coaxial fibers including a core layer and a cladding layer cladding the core layer, the core layer produced from the first spinning solution, the cladding layer produced from the second spinning solution.

Optionally, only the first spinning solution may be introduced into the electrospinning device, wherein solid fibers are produced and interwoven in the electrospinning process to form at least part of the vascular stent.

Optionally, the coaxial fibers may be interwoven into a first fiber layer, and the solid fibers may be interwoven into a second fiber layer, wherein the first and second fiber layers are stacked one on the other to form the vascular stent.

Optionally, a thickness of the first fiber layer may be 60% to 80% of a total thickness of the vascular stent.

Optionally, the biodegradable, water-insoluble polymer may include one of polylactic acid, polyglycolide acid, polycaprolactone, poly-p-dioxanone, poly(trimethylene carbonate), polyesteramide, polyhydroxyalkanoate, polyurethane and poly(lactic-co-glycolic) acid, or a mixture and/or copolymer of two or more thereof.

Optionally, the biodegradable, water-soluble polymer may include one of polyvinyl alcohol, polyethylene glycol, polyethylene oxide, polyvinyl pyrrolidone, poly(ethylene imine), hyaluronic acid, gelatin, collagen, sodium alginate and hyaluronic acid, or a mixture and/or copolymer of two or more thereof.

Optionally, the solvents may each include at least one of water, hexafluoroisopropanol, dichloromethane, trichloromethane, tetrahydrofuran, *N*,*N-*dimethylformamide, dimethyl sulfoxide, acetone, *n*-propyl acetate and *n*-butyl acetate.

Optionally, the method may further include:
subjecting the resulting vascular stent to heat treatment,
the heat treatment including:
   placing the vascular stent in a heating medium and heating, holding and cooling the heating medium.

Optionally, the heating medium may include one of air, ethanol, ethylene glycol and glycerol, or a mixture of two or more thereof.

The present invention also provides a method of fabricating a vascular stent system, which includes:
selecting a vascular stent fabricated in accordance with a method as defined above;
selecting a balloon and disposing the vascular stent over the balloon; and
placing the vascular stent into a crimping medium and crimping it so that it is crimped onto the balloon.

Optionally, the crimping medium may include one of air, ethanol, ethylene glycol and glycerol, or a mixture of two or more thereof.

The present invention also provides a vascular stent including a stent body including a first fiber layer and a second fiber layer, which are stacked one on the other,
the first fiber layer formed of interwoven coaxial fibers each including a core layer and a cladding layer, the core layer produced from a biodegradable, water-insoluble polymer, the cladding layer produced from a biodegradable, water-soluble polymer, the cladding layer cladding the core layer,
the second fiber layer formed of interwoven solid fibers produced from a biodegradable, water-insoluble polymer.

The present invention also provides a vascular stent system including a vascular stent as defined above and a balloon, the vascular stent disposed over and crimped onto the balloon.

During the delivery and expansion of a vascular stent, which is fabricated from biodegradable, water-insoluble and water-soluble polymers by electrospinning, as described above, within the human body, biodegradable, water-soluble components of fibers therein will dissolve in bodily fluids, facilitating relative sliding and dislocation movements of the remainder of the fibers, and hence enhancing the stent's expansion properties and overexpansion capabilities. On the other hand, the biodegradable, water-insoluble remainder is almost not affected, allowing the vascular stent to well maintain its mechanical properties. The use of the innovative electrospinning technique for biodegradable stent fabrication can overcome the critical challenge of poor overexpansion capabilities faced by biodegradable polymer stents, increasing the success of surgical procedures employing such stents.

### BRIEF DESCRIPTION OF THE DRAWING

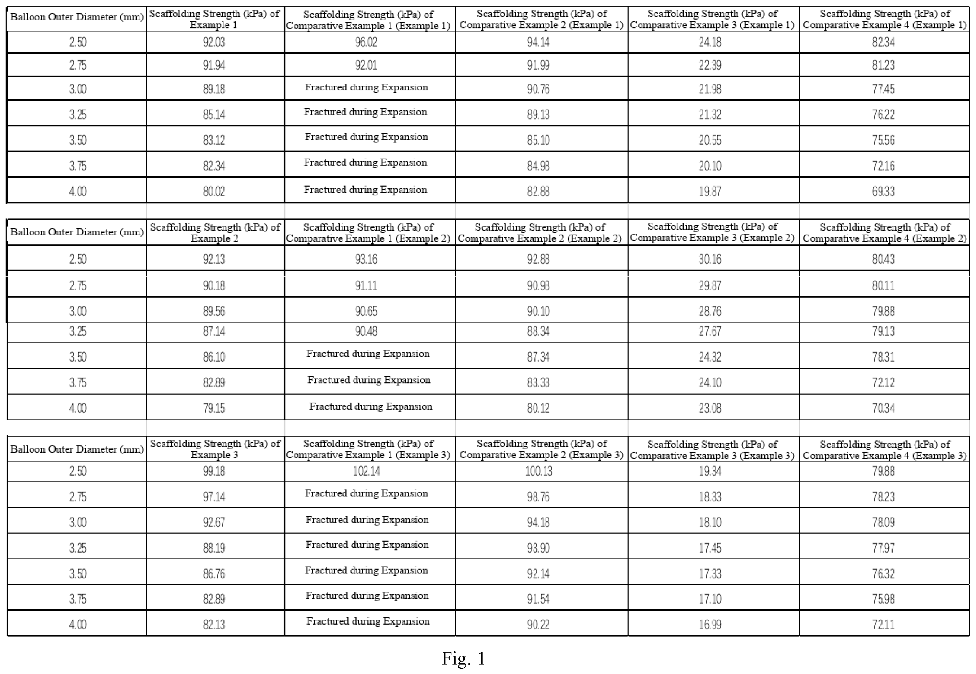

Fig. 1 shows a table listing scaffolding strength measurements of vascular stents prepared in accordance with examples of embodiments of the present invention.

### DETAILED DESCRIPTION

Vascular stents according to specific embodiments of the present invention will be described in greater detail below with reference to the accompanying drawing. From the following description, advantages and features of the present invention will become more apparent. Note that the figure is provided in a very simplified form not necessarily drawn to exact scale for the only purpose of facilitating easy and clear description of embodiments disclosed herein.

Embodiments disclosed herein provide vascular stents.

According to the present invention, vascular stents with improved radial scaffolding performance and overexpansion capabilities can be obtained from chosen materials in controlled proportions using novel electrospinning and heat treatment processes. Moreover, each vascular stent can be subjected to a crimping process to obtain a vascular stent system. The vascular stents provide both high scaffolding strength and enhanced expansion properties and can be made in various sizes, facilitating physicians' flexible choice of suitable stent sizes for different surgical procedures.

Suitable materials for the vascular stents include biodegradable, water-insoluble polymers and biodegradable, water-soluble polymers.

The vascular stents are each made from an assembly of interwoven fibers of one or more of the materials. The fibers are micro/nanofibers produced using electrospinning device. The fibers may be coaxial fibers, or solid fibers. As used herein, the term "interwoven" refers to mutual crossing and entanglement of fibers. In particular, the fibers may be interwoven, for example, using plain weaving, twill weaving, or another known weaving technique.

The solid fibers may be produced from one of the aforementioned biodegradable, water-insoluble polymers using a process including: dissolving the biodegradable, water-insoluble polymer in a solvent, obtaining a first spinning solution; introducing the first spinning solution into the electrospinning device; and obtaining the solid fibers by extrusion through a standard nozzle of the electrospinning device. The solid fibers may be made in a desired size by appropriately controlling process parameters for the electrospinning device, including relative molecular weight of the polymer, concentration of the solution, type of solvent, voltage, injection rate, tip-to-collector distance, ambient temperature and humidity, amongst others.

The coaxial fibers may be produced by co-extrusion of one of the aforementioned biodegradable, water-insoluble polymers and one of the biodegradable, water-soluble polymers. Each coaxial fiber includes a core layer and a cladding layer. The core layer is made from the biodegradable, water-insoluble polymer, and the cladding layer is made from the biodegradable, water-soluble polymer and clads the core layer on its exterior.

A process for producing the coaxial fibers may include: dissolving the biodegradable, water-insoluble polymer in a solvent, obtaining a first spinning solution for core-layer formation; and dissolving the biodegradable, water-soluble polymer in a solvent, obtaining a second spinning solution for cladding-layer formation. The co-extrusion may be accomplished using a coaxial electrospinning nozzle, which is a dual-channel composite nozzle. Such nozzles are known in the art, and further description thereof is omitted herein. The first and second spinning solutions are then introduced into the electrospinning device. Specifically, the second spinning solution is introduced into a cladding-layer channel of the coaxial electrospinning nozzle, and the first spinning solution is introduced into a core-layer channel of the coaxial electrospinning nozzle. Coaxial electrospinning is then initiated under preset parameters, including spinning voltage, core-layer and cladding-layer injection rates, tip-to-collector distance, ambient temperature and humidity, among others. The cladding- and core-layer solutions remain unmixed prior to spinning and solidification. As soon as a high-voltage electric field is applied, the second solution is drawn out, and the first solution is extruded simultaneously, causing the development of a composite Taylor cone at the nozzle. Finally, coaxial composite fibers each including a core layer cladded by a cladding layer are obtained. These are composite micro/nanofibers, in which the outer cladding layers are water-soluble, and the inner core layers are biodegradable. During the delivery and expansion of a vascular stent made of such coaxial fibers, the water-soluble outer layers of the fibers will dissolve in bodily fluids, resulting in increased fiber-to-fiber spacings, which facilitate their relative sliding and dislocation movements. This significantly enhances the stent's expansion properties.

The electrospinning device is equipped with a collector, which is a cylindrical mandrel of a given size and rotating at a given speed. The fibers are deposited onto a surface of the mandrel in a given manner, and finally collected in the form of a cylindrical layer.

A vascular stent may consist of interwoven coaxial fibers produced by the electrospinning device. Alternatively, a vascular stent may be constructed of alternating first and second fiber layers, which consist of solid and coaxial fibers alternately produced by the electrospinning device.

According to an embodiment disclosed herein, a vascular stent is included of a stack of multiple layers of fibers, including one or more first fiber layers consisting of interwoven coaxial fibers and one or more second fiber layers consisting of interwoven solid fibers.

There may be one or more first fiber layers and one or more second fiber layers alternating with the first fiber layers.

A vascular stent may include two fiber layers, for example, an inner first fiber layer and an outer second fiber layer, or an inner second fiber layer and an outer first fiber layer.

A vascular stent may include three fiber layers, for example, an inner first fiber, an intermediate second fiber layer and an outer first fiber layer, or an inner second fiber, an intermediate first fiber layer and an outer second fiber layer.

Such a vascular stent may be fabricated using a layer-wise deposition method including: depositing fibers from the electrospinning device onto the mandrel surface in a given manner, thereby forming an innermost fiber layer; depositing fibers from the electrospinning device onto the exterior of the innermost fiber layer in a given manner, thereby forming a second innermost fiber layer; and so forth.

Of course, vascular stents according to other embodiments not described herein may include more fiber layers.

In the processes discussed above, preferably the first and second spinning solutions may each have a concentration lying between 1% and 20%. Preferably, the biodegradable, water-insoluble and water-soluble polymers may each have a relative molecular weight ranging from 1,000 to 100,0000. The processes may be carried out at a spinning voltage of 1 to 50 kV, an injection rate of 0.01 to 1 mm/min, a tip-to-collector distance of 50 to 300 mm, an ambient temperature of 18 to 30 °C and an ambient humidity of 25% to 60%. The collector may have an outer diameter of 0.1 to 5 mm and be rotated at a speed of 5 to 5,000 rpm. The size of the coaxial electrospinning nozzle may be 18G/14G, 19G/15G, 22G/17G, 25G/18G, 30G/21G, etc.

In the above processes, the biodegradable, water-insoluble polymer may include one of polylactic acid (PLA), polyglycolide acid (PGA), polycaprolactone (PCL), poly-p-dioxanone (PPDO), poly(trimethylene carbonate) (PTMC), polyesteramide (PEA), polyhydroxyalkanoate (PHA), polyurethane (PU) and poly(lactic-co-glycolic acid) 82:18 (PLGA8218), or a mixture and/or copolymer of two or more thereof.

In the above processes, the biodegradable, water-soluble polymer may include one of polyvinyl alcohol (PVA), polyethylene glycol (PEG), polyethylene oxide (PEO), polyvinyl pyrrolidone (PVP), poly(ethylene imine) (PEI), hyaluronic acid (HA), gelatin (GEL), collagen (COL) and sodium alginate (SA), or a mixture and/or copolymer of two or more thereof.

In the above processes, each of the biodegradable, water-insoluble and water-soluble polymers is dissolved in a solvent, which may include at least one of water, hexafluoroisopropanol (HFIP), dichloromethane (DCM), trichloromethane (TCM), tetrahydrofuran (THF), N,N-dimethylformamide (DMF), dimethyl sulfoxide (DMSO), acetone, *n*-propyl acetate (NPAC) and *n*-butyl acetate (NBAC).

During the delivery and expansion of a vascular stent, which is fabricated from biodegradable, water-insoluble and water-soluble polymers by coaxial electrospinning, as described above, within the human body, the outer water-soluble layers of the coaxial fibers will dissolve in bodily fluids, facilitating relative sliding and dislocation movements of the remainder of the fibers and hence enhancing the stent's expansion properties and overexpansion capabilities. On the other hand, the remaining biodegradable core layers are almost not affected, allowing the vascular stent to well maintain its mechanical properties. The use of this innovative coaxial electrospinning technique for biodegradable stent fabrication can overcome the critical challenge of poor overexpansion capabilities faced by biodegradable polymer stents, increasing the success of surgical procedures employing such stents.

In practical applications, vascular stents with desired expansion properties and scaffolding performance can be obtained at appropriate ratios of the materials. Preferably, in each vascular stent, first fiber layer(s) may account for 60% to 80%, and therefore second fiber layer(s) for 20% to 40%, of a total thickness of the vascular stent.

On the basis of vascular stents as defined above, embodiments disclosed herein also provide vascular stent systems and methods of fabrication thereof. Such a vascular stent system includes a vascular stent, as discussed above, and a balloon, and is obtained by crimping the vascular stent onto the balloon. The crimping is necessary before the vascular stent can be delivered into and within the human body. The balloon is used to expand the vascular stent after the vascular stent system is delivered to a target site within the human body. The crimping may be accomplished as is conventional in the art, and the balloon may be implemented as a conventional one.

The vascular stent system may be obtained according to a method including fabricating and crimping the vascular stent. The fabrication of the vascular stent may include: producing a fiber assembly into the vascular stent by electrospinning device; and subjecting the vascular stent to heat treatment. The heat treatment is optional and may be omitted depending on internal stress of the vascular stent being fabricated.

The vascular stent is made from fibers collected on the surface of a collector as a result of stretching and solidification of a jet created in an electrospinning. Since significant internal stress is generated during the electrospinning process in the vascular stent being fabricated, which is unfavorable for its mechanical properties, the vascular stent is typically subjected to the heat treatment for eliminating its internal stress. Thus, the heat treatment and crimping can maintain and enhance the mechanical properties of the resulting electrospun stent.

The heat treatment may include: selecting a heating mandrel; disposing the vascular stent over the heating mandrel immersed in a heating medium; and heating, holding and cooling the heating medium. The heating, holding and cooling step may be repeated multiple times. The heating medium may include one of air, ethanol, ethylene glycol and glycerol, or a mixture of two or more thereof. Control parameters for the heat treatment may include, among others, initial temperature, heating rate, holding duration, cooling rate, final temperature and number of heating, holding and cooling cycles.

Specifically, the initial temperature may be in the range of 25 °C to 150 °C, the heating rate of 0.1 °C/min to 10 °C/min, the holding duration of 1 minute to 120 minutes, the cooling rate of 0.1 °C/min to 10 °C/min, the final temperature of 25 °C to 150 °C, and the number of heating, holding and cooling cycles of 1 to 5.

The crimping may follow the heat treatment, thereby obtaining the vascular stent system.

The crimping involves crimping the vascular stent under certain parameters onto the balloon where it assumes a size suitable for its delivery within the human body.

To ensure good mechanical properties of the vascular stent, the crimping may be immersed and crimped in a crimping medium. In this process, parameters including crimping rate, medium temperature and the like may be controlled to allow the crimping of the vascular stent to be accomplished while ensuring good mechanical properties of the vascular stent.

The crimping parameters may include type of crimping medium, crimping rate, temperature of the crimping medium, crimping duration and crimping temperature, amongst other things.

The crimping medium may include one of air, ethanol, ethylene glycol and glycerol, or a mixture of two or more thereof.

Specifically, the crimping rate may range from 0.01 mm/s to 20 mm/s, the temperature of the crimping medium from 25 °C to 80 °C, the crimping duration from 0 to 999 s, and the crimping temperature from 25 °C to 80 °C.

In practice, the vascular stent that has experienced the heat treatment may be placed in a crimper and then crimped under preset parameters. The crimping may be accomplished using any suitable conventional technique and, therefore, need not be described in further detail herein. However, the present invention differs therefrom in the use of and options for the crimping medium.

As noted above, vascular stents may be fabricated from biodegradable, water-insoluble and water-soluble polymers using coaxial electrospinning and then subjected to heat treatment and crimping processes, thereby obtaining vascular stent systems with good overexpansion capabilities and good scaffolding performance.

The fabrication of such vascular stents and vascular stent systems will be further described below by way of specific examples.

Fifteen examples are given below, which are categorized into three groups:
1) Example 1 and corresponding Comparative Examples 1, 2 3 and 4;
2) Example 2 and corresponding Comparative Examples 1, 2 3 and 4; and
3) Example 3 and corresponding Comparative Examples 1, 2 3 and 4.

In Comparative Examples 1 that respectively correspond to Example 1, 2 and 3, vascular stents were fabricated simply from solid fibers made of biodegradable, water-insoluble polymer(s), as conventional vascular stents for comparative demonstration of mechanical and expansion properties. In each of the other examples, a vascular stent was prepared in accordance with a method embodying the present invention.

### Example 1

### S1: Producing Fiber Assembly in the Shape of Vascular Stent

PLA with a molecular weight of 250,000 to 350,000 was dissolved in a solvent obtained by mixing TCM and DMF at a volume ratio of 7: 3, obtaining a 6-10% (w/w) first solution. PEG with a molecular weight of 2,000 to 8,000 was dissolved in water, obtaining a 1-4% (w/w) second solution. The first solution was introduced into a core-layer channel of a coaxial electrospinning nozzle, and the second solution was introduced into a cladding-layer channel of the coaxial electrospinning nozzle.

An electrospinning process was performed using electrospinning device under the following parameters: 22G/17G coaxial electrospinning nozzle; spinning voltage of 6-8 kV; cladding-layer injection rate of 0.20-0.30 mm/min; core-layer injection rate of 0.10-0.20 mm/min; tip-to-collector distance of 80-100 mm; ambient temperature of 23±1 °C; and ambient humidity of 30±5%. The collector had an outer diameter of 2.5 mm and was rotated at a speed of 1,000-1,500 rpm.

A first fiber layer was deposited onto the collector in the shape of a vascular stent with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: 2.5 mm heating mandrel; initial temperature of 25-28 °C; heating rate of 1.0-1.5 °C/min; holding duration of 10-15 min; cooling rate of 5-6 °C/min; final temperature of 25-28 °C; and one heating, holding and cooling cycle.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.1-0.2 mm/s; temperature of the crimping medium of 60-70 °C; crimping duration of 300 s; and crimping temperature of 60-70 °C.

### Comparative Example 1 (Example 1)

In this Comparative Example, a vascular stent was fabricated simply from the first solution of Example 1. Therefore, the vascular stent consisted of solid fibers produced from the biodegradable, water-insoluble polymer.

### S1: Producing Fiber Assembly in the Shape of Vascular Stent

PLA with a molecular weight of 250,000 to 350,000 was dissolved in a solvent obtained by mixing TCM and DMF at a volume ratio of 7: 3, obtaining a 6-10% (w/w) first solution. An electrospinning process was performed using electrospinning device under the following parameters: 22G electrospinning nozzle; spinning voltage of 6-8 kV; injection rate of 0.10-0.20 mm/min; tip-to-collector distance of 80-100 mm; ambient temperature of 23±1 °C; and ambient humidity of 30±5%. The collector had a size of 2.5 mm and was rotated at a speed of 1,000-1,500 rpm.

A second fiber layer was deposited onto the collector in the shape of a vascular stent with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: 2.5 mm heating mandrel; initial temperature of 25-28 °C; heating rate of 1.0-1.5 °C/min; holding duration of 10-15 min; cooling rate of 5-6 °C/min; final temperature of 25-28 °C; and one heating, holding and cooling cycle.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.1-0.2 mm/s; temperature of the crimping medium of 60-70 °C; crimping duration of 300 s; and crimping temperature of 60-70 °C.

### Comparative Example 2 (Example 1)

### S1: Producing Fiber Assembly in the Shape of Vascular Stent

PLA with a molecular weight of 250,000 to 350,000 was dissolved in a solvent obtained by mixing TCM and DMF at a volume ratio of 7: 3, obtaining a 6-10% (w/w) first solution. PEG with a molecular weight of 2,000 to 8,000 was dissolved in water, obtaining a 1-4% (w/w) second solution. An electrospinning process was performed using electrospinning device under the following parameters: 22G/17G coaxial electrospinning nozzle; spinning voltage of 6-8 kV; cladding-layer injection rate of 0.20-0.30 mm/min; core-layer injection rate of 0.10-0.20 mm/min; tip-to-collector distance of 80-100 mm; ambient temperature of 23±1 °C; and ambient humidity of 30±5%. The collector had an outer diameter of 2.5 mm and was rotated at a speed of 1,000-1,500 rpm. A first fiber layer was deposited to a thickness of 20 µm onto the collector.

PLA with a molecular weight of 250,000 to 350,000 was dissolved in a solvent obtained by mixing TCM and DMF at a volume ratio of 7: 3, obtaining a 6-10% (w/w) first solution. An electrospinning process was performed using the electrospinning device under the following parameters: 22G electrospinning nozzle; spinning voltage of 6-8 kV; injection rate of 0.10-0.20 mm/min; tip-to-collector distance of 80-100 mm; ambient temperature of 23±1 °C; and ambient humidity of 30±5%. The collector had an outer diameter of 2.5 mm and was rotated at a speed of 1,000-1,500 rpm. A second fiber layer was deposited to a thickness of 80 µm onto the first fiber layer on the collector.

Thus, a vascular stent was obtained with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: 2.5 mm heating mandrel; initial temperature of 25-28 °C; heating rate of 1.0-1.5 °C/min; holding duration of 10-15 min; cooling rate of 5-6 °C/min; final temperature of 25-28 °C; and one heating, holding and cooling cycle.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.1-0.2 mm/s; temperature of the crimping medium of 60-70 °C; crimping duration of 300 s; and crimping temperature of 60-70 °C.

### Comparative Example 3 (Example 1)

### S1: Producing Fiber Assembly in the Shape of Vascular Stent

PLA with a molecular weight of 250,000 to 350,000 was dissolved in a solvent obtained by mixing TCM and DMF at a volume ratio of 7: 3, obtaining a 6-10% (w/w) first solution. PEG with a molecular weight of 2,000 to 8,000 was dissolved in water, obtaining a 1-4% (w/w) second solution. An electrospinning process was performed using electrospinning device under the following parameters: 22G/17G coaxial electrospinning nozzle; spinning voltage of 6-8 kV; cladding-layer injection rate of 0.20-0.30 mm/min; core-layer injection rate of 0.10-0.20 mm/min; tip-to-collector distance of 80-100 mm; ambient temperature of 23±1 °C; and ambient humidity of 30±5%. The collector had an outer diameter of 2.5 mm and was rotated at a speed of 1,000-1,500 rpm. A first fiber layer was deposited to a thickness of 20 µm onto the collector.

PLA with a molecular weight of 250,000 to 350,000 was dissolved in a solvent obtained by mixing TCM and DMF at a volume ratio of 7: 3, obtaining a 6-10% (w/w) solution. An electrospinning process was performed using the electrospinning device under the following parameters: 22G electrospinning nozzle; spinning voltage of 6-8 kV; injection rate of 0.10-0.20 mm/min; tip-to-collector distance of 80-100 mm; ambient temperature of 23±1 °C; and ambient humidity of 30±5%. The collector had an outer diameter of 2.5 mm and was rotated at a speed of 1,000-1,500 rpm. A second fiber layer was deposited to a thickness of 80 µm onto the first fiber layer on the collector.

Thus, a vascular stent was obtained with a total thickness of 100 µm.

### S2: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.1-0.2 mm/s; temperature of the crimping medium of 60-70 °C; crimping duration of 300 s; and crimping temperature of 60-70 °C.

### Comparative Example 4 (Example 1)

### S1: Producing Fiber Assembly in the Shape of Vascular Stent

PLA with a molecular weight of 250,000 to 350,000 was dissolved in a solvent obtained by mixing TCM and DMF at a volume ratio of 7: 3, obtaining a 6-10% (w/w) first solution. PEG with a molecular weight of 2,000 to 8,000 was dissolved in water, obtaining a 1-4% (w/w) second solution. An electrospinning process was performed using electrospinning device under the following parameters: 22G/17G coaxial electrospinning nozzle; spinning voltage of 6-8 kV; cladding-layer injection rate of 0.20-0.30 mm/min; core-layer injection rate of 0.10-0.20 mm/min; tip-to-collector distance of 80-100 mm; ambient temperature of 23±1 °C; and ambient humidity of 30±5%. The collector had an outer diameter of 2.5 mm and was rotated at a speed of 1,000-1,500 rpm. A first fiber layer was deposited to a thickness of 20 µm onto the collector.

PLA with a molecular weight of 250,000 to 350,000 was dissolved in a solvent obtained by mixing TCM and DMF at a volume ratio of 7: 3, obtaining a 6-10% (w/w) solution. An electrospinning process was performed using the electrospinning device under the following parameters: 22G electrospinning nozzle; spinning voltage of 6-8 kV; injection rate of 0.10-0.20 mm/min; tip-to-collector distance of 80-100 mm; ambient temperature of 23±1 °C; and ambient humidity of 30±5%. The collector had an outer diameter of 2.5 mm and was rotated at a speed of 1,000-1,500 rpm. A second fiber layer was deposited to a thickness of 80 µm onto the first fiber layer on the collector.

Thus, a vascular stent was obtained with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: 2.5 mm heating mandrel; initial temperature of 25-28 °C; heating rate of 1.0-1.5 °C/min; holding duration of 10-15 min; cooling rate of 5-6 °C/min; final temperature of 25-28 °C; and one heating, holding and cooling cycle.

### Example 2

### S1: Producing Fiber Assembly in the Shape of Vascular Stent

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. PVA with a molecular weight of 4,000 to 10,000 was dissolved in water, obtaining an 8-11% (w/w) second solution. An electrospinning process was performed using electrospinning device under the following parameters: 19G/15G coaxial electrospinning nozzle; spinning voltage of 12-14 kV; cladding-layer injection rate of 0.45-0.55 mm/min; core-layer injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm.

A first fiber layer was deposited onto the collector in the shape of a vascular stent with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: balloon with an outer diameter of 1.5 mm as the heating mandrel; initial temperature of 27-30 °C; heating rate of 0.5-1.0 °C/min; holding duration of 60-65 min; cooling rate of 9-10 °C/min; and final temperature of 27-30 °C; and three heating, holding and cooling cycles.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.2-0.3 mm/s; temperature of the crimping medium of 25-35 °C; crimping duration of 120 s; and crimping temperature of 25-35 °C.

### Comparative Example 1 (Example 2)

In this Comparative Example, a vascular stent was fabricated simply from the first solution of Example 2. Therefore, the vascular stent consisted of solid fibers produced from the biodegradable, water-insoluble polymers.

### S1: Producing Micro/Nanofiber Assembly

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. An electrospinning process was performed using electrospinning device under the following parameters: 19G electrospinning nozzle; spinning voltage of 12-14 kV; injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A second fiber layer was deposited onto the collector in the shape of a vascular stent with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: balloon with an outer diameter of 1.5 mm as the heating mandrel; initial temperature of 27-30 °C; heating rate of 0.5-1.0 °C/min; holding duration of 60-65 min; cooling rate of 9-10 °C/min; and final temperature of 27-30 °C; and three heating, holding and cooling cycles.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.2-0.3 mm/s; temperature of the crimping medium of 25-35 °C; crimping duration of 120 s; and crimping temperature of 25-35 °C.

### Comparative Example 2 (Example 2)

### S1: Producing Fiber Assembly in the Shape of Vascular Stent

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. PVA with a molecular weight of 4,000 to 10,000 was dissolved in water, obtaining an 8-11% (w/w) second solution. An electrospinning process was performed using electrospinning device under the following parameters: 19G/15G coaxial electrospinning nozzle; spinning voltage of 12-14 kV; cladding-layer injection rate of 0.45-0.55 mm/min; core-layer injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A first fiber layer was deposited to a thickness of 20 µm onto the collector.

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. An electrospinning process was performed using the electrospinning device under the following parameters: 19G electrospinning nozzle; spinning voltage of 12-14 kV; injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A second fiber layer was deposited to a thickness of 60 µm onto the first fiber layer on the collector.

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. PVA with a molecular weight of 4,000 to 10,000 was dissolved in water, obtaining an 8-11% (w/w) second solution. An electrospinning process was performed using the electrospinning device under the following parameters: 19G/15G coaxial electrospinning nozzle; spinning voltage of 12-14 kV; cladding-layer injection rate of 0.45-0.55 mm/min; core-layer injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A first fiber layer was deposited to a thickness of 20 µm onto the second fiber layer on the collector.

Thus, a vascular stent was obtained as a three-layer composite structure consisting of an inner first fiber layer, an intermediate second fiber layer and an outer first fiber layer and having a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: balloon with an outer diameter of 1.5 mm as the heating mandrel; initial temperature of 27-30 °C; heating rate of 0.5-1.0 °C/min; holding duration of 60-65 min; cooling rate of 9-10 °C/min; and final temperature of 27-30 °C; and three heating, holding and cooling cycles.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.2-0.3 mm/s; temperature of the crimping medium of 25-35 °C; crimping duration of 120 s; and crimping temperature of 25-35 °C.

### Comparative Example 3 (Example 2)

### S1: Producing Fiber Assembly in the Shape of Vascular Stent

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. PVA with a molecular weight of 4,000 to 10,000 was dissolved in water, obtaining an 8-11% (w/w) second solution. An electrospinning process was performed using electrospinning device under the following parameters: 19G/15G coaxial electrospinning nozzle; spinning voltage of 12-14 kV; cladding-layer injection rate of 0.45-0.55 mm/min; core-layer injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A first fiber layer was deposited to a thickness of 20 µm onto the collector.

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. An electrospinning process was performed using the electrospinning device under the following parameters: 19G electrospinning nozzle; spinning voltage of 12-14 kV; injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A second fiber layer was deposited to a thickness of 60 µm onto the first fiber layer on the collector.

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. PVA with a molecular weight of 4,000 to 10,000 was dissolved in water, obtaining an 8-11% (w/w) second solution. An electrospinning process was performed using the electrospinning device under the following parameters: 19G/15G coaxial electrospinning nozzle; spinning voltage of 12-14 kV; cladding-layer injection rate of 0.45-0.55 mm/min; core-layer injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A first fiber layer was deposited to a thickness of 20 µm onto the second fiber layer on the collector.

Thus, a vascular stent was obtained as a three-layer composite structure consisting of an inner first fiber layer, an intermediate second fiber layer and an outer first fiber layer and having a total thickness of 100 µm.

### S2: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: air as the crimping medium; crimping rate of 0.2-0.3 mm/s; temperature of the crimping medium of 25-35 °C; crimping duration of 120 s; and crimping temperature of 25-35 °C.

### Comparative Example 4 (Example 2)

### S1: Producing Fiber Assembly in the Shape of Vascular Stent

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. PVA with a molecular weight of 4,000 to 10,000 was dissolved in water, obtaining an 8-11% (w/w) second solution. An electrospinning process was performed using electrospinning device under the following parameters: 19G/15G coaxial electrospinning nozzle; spinning voltage of 12-14 kV; cladding-layer injection rate of 0.45-0.55 mm/min; core-layer injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A first fiber layer was deposited to a thickness of 20 µm onto the collector.

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. An electrospinning process was performed using the electrospinning device under the following parameters: 19G electrospinning nozzle; spinning voltage of 12-14 kV; injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A second fiber layer was deposited to a thickness of 60 µm onto the first fiber layer on the collector.

PLA with a molecular weight of 350,000 to 450,000 and PCL with a molecular weight of 70,000 to 90,000 were dissolved at a weight ratio of 9: 1 in HFIP, obtaining a 12-16% (w/w) first solution. PVA with a molecular weight of 4,000 to 10,000 was dissolved in water, obtaining an 8-11% (w/w) second solution. An electrospinning process was performed using the electrospinning device under the following parameters: 19G/15G coaxial electrospinning nozzle; spinning voltage of 12-14 kV; cladding-layer injection rate of 0.45-0.55 mm/min; core-layer injection rate of 0.15-0.25 mm/min; tip-to-collector distance of 120-140 mm; ambient temperature of 22±1 °C; and ambient humidity of 35±5%. The collector had an outer diameter of 2.5 mm and was rotated at a speed of 2,000-2,500 rpm. A first fiber layer was deposited to a thickness of 20 µm onto the second fiber layer on the collector.

Thus, a vascular stent was obtained as a three-layer composite structure consisting of an inner first fiber layer, an intermediate second fiber layer and an outer first fiber layer and having a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: balloon with an outer diameter of 1.5 mm as the heating mandrel; initial temperature of 27-30 °C; heating rate of 0.5-1.0 °C/min; holding duration of 60-65 min; cooling rate of 9-10 °C/min; and final temperature of 27-30 °C; and three heating, holding and cooling cycles.

### Example 3

### S1: Producing Fiber Assembly in the Shape of Vascular Stent

PLGA8218 with a molecular weight of 200,000-300,000 was dissolved in HFIP, obtaining a 4-6% (w/w) first solution. HA with a molecular weight of 30,000 to 45,000 was dissolved in water, obtaining a 2-5% (w/w) second solution. An electrospinning process was performed using electrospinning device under the following parameters: 18G/14G coaxial electrospinning nozzle; spinning voltage of 20-22 kV; cladding-layer injection rate of 0.10-0.15 mm/min; core-layer injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm.

A first fiber layer was deposited onto the collector in the shape of a vascular stent with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: heating mandrel with an outer diameter of 2.5 mm; initial temperature of 19-22 °C; heating rate of 2.0-2.5 °C/min; holding duration of 5-10 min; cooling rate of 7-8 °C/min; and final temperature of 19-22 °C; and two heating, holding and cooling cycles.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: ethanol and ethylene glycol mixed at a volume ratio of 7: 3 as the crimping medium; crimping rate of 0.02-0.12 mm/s; temperature of the crimping medium of 45-55 °C; crimping duration of 30 s; and crimping temperature of 45-55 °C.

### Comparative Example 1 (Example 3)

In this Comparative Example, a vascular stent was fabricated simply from the first solution of Example 3. Therefore, the vascular stent consisted of solid fibers produced from the biodegradable, water-insoluble polymer.

### S1: Producing Micro/Nanofiber Assembly

PLGA8218 with a molecular weight of 200,000-300,000 was dissolved in HFIP, obtaining a 4-6% (w/w) first solution. An electrospinning process was performed using electrospinning device under the following parameters: 18G electrospinning nozzle; spinning voltage of 20-22 kV; injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm.

A second fiber layer was deposited onto the collector in the shape of a vascular stent with a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: heating mandrel with an outer diameter of 2.5 mm; initial temperature of 19-22 °C; heating rate of 2.0-2.5 °C/min; holding duration of 5-10 min; cooling rate of 7-8 °C/min; and final temperature of 19-22 °C; and two heating, holding and cooling cycles.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: ethanol and ethylene glycol mixed at a volume ratio of 7: 3 as the crimping medium; crimping rate of 0.02-0.12 mm/s; temperature of the crimping medium of 45-55 °C; crimping duration of 30 s; and crimping temperature of 45-55 °C.

### Comparative Example 2 (Example 3)

### S1: Producing Fiber Assembly in the Shape of Vascular Stent

PLGA8218 with a molecular weight of 200,000-300,000 was dissolved in HFIP, obtaining a 4-6% (w/w) first solution. HA with a molecular weight of 30,000 to 45,000 was dissolved in water, obtaining a 2-5% (w/w) second solution. An electrospinning process was performed using electrospinning device under the following parameters: 18G/14G coaxial electrospinning nozzle; spinning voltage of 20-22 kV; cladding-layer injection rate of 0.10-0.15 mm/min; core-layer injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A first fiber layer was deposited to a thickness of 10 µm onto the collector.

PLGA8218 with a molecular weight of 200,000-300,000 was dissolved in HFIP, obtaining a 4-6% (w/w) solution. An electrospinning process was performed using the electrospinning device under the following parameters: 18G electrospinning nozzle; spinning voltage of 20-22 kV; injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A second fiber layer was deposited to a thickness of 80 µm onto the first fiber layer on the collector.

PLGA8218 with a molecular weight of 200,000-300,000 was dissolved in HFIP, obtaining a 4-6% (w/w) first solution. HA with a molecular weight of 30,000 to 45,000 was dissolved in water, obtaining a 2-5% (w/w) second solution. An electrospinning process was performed using electrospinning device under the following parameters: 18G/14G coaxial electrospinning nozzle; spinning voltage of 20-22 kV; cladding-layer injection rate of 0.10-0.15 mm/min; core-layer injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A first fiber layer was deposited to a thickness of 10 µm onto the second fiber layer on the collector.

Thus, a vascular stent was obtained as a three-layer composite structure consisting of an inner first fiber layer, an intermediate second fiber layer and an outer first fiber layer and having a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: heating mandrel with an outer diameter of 2.5 mm; initial temperature of 19-22 °C; heating rate of 2.0-2.5 °C/min; holding duration of 5-10 min; cooling rate of 7-8 °C/min; and final temperature of 19-22 °C; and two heating, holding and cooling cycles.

### S3: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: ethanol and ethylene glycol mixed at a volume ratio of 7: 3 as the crimping medium; crimping rate of 0.02-0.12 mm/s; temperature of the crimping medium of 45-55 °C; crimping duration of 30 s; and crimping temperature of 45-55 °C.

### Comparative Example 3 (Example 3)

### S1: Producing Fiber Assembly in the Shape of Vascular Stent

PLGA8218 with a molecular weight of 200,000-300,000 was dissolved in HFIP, obtaining a 4-6% (w/w) first solution. HA with a molecular weight of 30,000 to 45,000 was dissolved in water, obtaining a 2-5% (w/w) second solution. An electrospinning process was performed using electrospinning device under the following parameters: 18G/14G coaxial electrospinning nozzle; spinning voltage of 20-22 kV; cladding-layer injection rate of 0.10-0.15 mm/min; core-layer injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A first fiber layer was deposited to a thickness of 10 µm onto the collector.

PLGA8218 with a molecular weight of 200,000-300,000 was dissolved in HFIP, obtaining a 4-6% (w/w) solution. An electrospinning process was performed using the electrospinning device under the following parameters: 18G electrospinning nozzle; spinning voltage of 20-22 kV; injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A second fiber layer was deposited to a thickness of 80 µm onto the first fiber layer on the collector.

PLGA8218 with a molecular weight of 200,000-300,000 was dissolved in HFIP, obtaining a 4-6% (w/w) first solution. HA with a molecular weight of 30,000 to 45,000 was dissolved in water, obtaining a 2-5% (w/w) second solution. An electrospinning process was performed using electrospinning device under the following parameters: 18G/14G coaxial electrospinning nozzle; spinning voltage of 20-22 kV; cladding-layer injection rate of 0.10-0.15 mm/min; core-layer injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A first fiber layer was deposited to a thickness of 10 µm onto the second fiber layer on the collector.

Thus, a vascular stent was obtained as a three-layer composite structure consisting of an inner first fiber layer, an intermediate second fiber layer and an outer first fiber layer and having a total thickness of 100 µm.

### S2: Crimping

The vascular stent was crimped onto a balloon so as to assume a size suitable for its delivery within the human body. The crimping was conducted within a crimping medium in order to ensure good mechanical properties of the vascular stent.

The crimping was performed under the following parameters: ethanol and ethylene glycol mixed at a volume ratio of 7: 3 as the crimping medium; crimping rate of 0.02-0.12 mm/s; temperature of the crimping medium of 45-55 °C; crimping duration of 30 s; and crimping temperature of 45-55 °C.

### Comparative Example 4 (Example 3)

### S1: Producing Fiber Assembly in the Shape of Vascular Stent

PLGA8218 with a molecular weight of 200,000-300,000 was dissolved in HFIP, obtaining a 4-6% (w/w) first solution. HA with a molecular weight of 30,000 to 45,000 was dissolved in water, obtaining a 2-5% (w/w) second solution. An electrospinning process was performed using electrospinning device under the following parameters: 18G/14G coaxial electrospinning nozzle; spinning voltage of 20-22 kV; cladding-layer injection rate of 0.10-0.15 mm/min; core-layer injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A first fiber layer was deposited to a thickness of 10 µm onto the collector.

PLGA8218 with a molecular weight of 200,000-300,000 was dissolved in HFIP, obtaining a 4-6% (w/w) solution. An electrospinning process was performed using the electrospinning device under the following parameters: 18G electrospinning nozzle; spinning voltage of 20-22 kV; injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A second fiber layer was deposited to a thickness of 80 µm onto the first fiber layer on the collector.

PLGA8218 with a molecular weight of 200,000-300,000 was dissolved in HFIP, obtaining a 4-6% (w/w) first solution. HA with a molecular weight of 30,000 to 45,000 was dissolved in water, obtaining a 2-5% (w/w) second solution. An electrospinning process was performed using electrospinning device under the following parameters: 18G/14G coaxial electrospinning nozzle; spinning voltage of 20-22 kV; cladding-layer injection rate of 0.10-0.15 mm/min; core-layer injection rate of 0.05-0.15 mm/min; tip-to-collector distance of 60-80 mm; ambient temperature of 25±1 °C; and ambient humidity of 45±5%. The collector had an outer diameter of 2.5 mm and was rotated at a speed of 4,500-5,000 rpm. A first fiber layer was deposited to a thickness of 10 µm onto the second fiber layer on the collector.

Thus, a vascular stent was obtained as a three-layer composite structure consisting of an inner first fiber layer, an intermediate second fiber layer and an outer first fiber layer and having a total thickness of 100 µm.

### S2: Heat Treatment

The vascular stent was disposed over a heating mandrel placed in air, and the heating medium was then subjected to a heating, holding and cooling cycle. The heating, holding and cooling cycle could be repeated multiple times.

Heat treatment was carried out under the following parameters: heating mandrel with an outer diameter of 2.5 mm; initial temperature of 19-22 °C; heating rate of 2.0-2.5 °C/min; holding duration of 5-10 min; cooling rate of 7-8 °C/min; and final temperature of 19-22 °C; and two heating, holding and cooling cycles.

The above examples are categorized into three groups: 1) Example 1 and corresponding Comparative Examples 1, 2 3 and 4; 2) Example 2 and corresponding Comparative Examples 1, 2 3 and 4; and 3) Example 3 and corresponding Comparative Examples 1, 2 3 and 4.

The vascular stents fabricated in Comparative Examples 1 that respectively correspond to Example 1, 2 and 3 all consist of solid fibers made of only biodegradable, water-insoluble polymer(s). These are conventional vascular stents for comparison.

As shown in Fig. 1, the vascular stents fabricated in the above examples were expanded with balloons of different outer diameters including 2.50, 2.75, 3.00, 3.25, 3.50, 3.75 and 4.00 mm, and observed during the expansion for any signs of fracture. Further, radial strength of each vascular stent that was successfully expanded was tested in accordance with the standard YY/T 0663.2-2016.

As can be seen from the data of Fig. 1, the coaxially electrospun vascular stents exhibited substantially comparable scaffolding strength to the conventional electrospun vascular stents (Comparative Examples 1 that respectively correspond to Example 1, 2 and 3).

Various levels of fracture were observed in the conventional electrospun vascular stents (Comparative Examples 1 that respectively correspond to Example 1, 2 and 3) during expansion, while the coaxially electrospun vascular stents exhibited much better expansion properties while maintaining radial strength within a desirable range, facilitating physicians' flexible choice of suitable stent sizes for different surgical procedures. Further, it has been demonstrated that the heat treatment has a significant impact on the stents' scaffolding performance.

The description presented above is merely that of a few preferred embodiments of the present invention and does not limit the scope thereof in any sense. Any and all changes and modifications made by those of ordinary skill in the art based on the above teachings fall within the scope as defined in the appended claims.

## Claims

1. A method of fabricating a vascular stent, comprising:
preparing a first spinning solution by dissolving a biodegradable, water-insoluble polymer in a solvent and preparing a second spinning solution by dissolving a biodegradable, water-soluble polymer in a solvent; and
introducing the first spinning solution and the second spinning solution to an electrospinning device and performing an electrospinning process, thereby forming the vascular stent.

2. The method of fabricating the vascular stent of claim 1, wherein the first spinning solution and the second spinning solution are simultaneously introduced into the electrospinning device, wherein coaxial fibers are produced and interwoven in the electrospinning process to form at least part of the vascular stent,
each of the coaxial fibers comprising a core layer and a cladding layer cladding the core layer, the core layer produced from the first spinning solution, the cladding layer produced from the second spinning solution.

3. The method of fabricating the vascular stent of claim 2, wherein only the first spinning solution is introduced into the electrospinning device, wherein solid fibers are produced and interwoven in the electrospinning process to form at least part of the vascular stent.

4. The method of fabricating the vascular stent of claim 3, wherein the coaxial fibers are interwoven into a first fiber layer and the solid fibers are interwoven into a second fiber layer, and wherein the first and second fiber layers are stacked one on the other to form the vascular stent.

5. The method of fabricating the vascular stent of claim 4, wherein a thickness of the first fiber layer is 60% to 80% of a total thickness of the vascular stent.

6. The method of fabricating the vascular stent of claim 4, wherein there are one or more of the first fiber layers and one or more of the second fiber layers, and wherein the first fiber layers alternate with the second fiber layers.

7. The method of fabricating the vascular stent of claim 1, wherein the biodegradable, water-insoluble polymer comprises one of polylactic acid, polyglycolide acid, polycaprolactone, poly-p-dioxanone, poly(trimethylene carbonate), polyesteramide, polyhydroxyalkanoate, polyurethane and poly(lactic-co-glycolic) acid, or a mixture and/or copolymer of two or more thereof.

8. The method of fabricating the vascular stent of claim 1, wherein the biodegradable, water-soluble polymer comprises one of polyvinyl alcohol, polyethylene glycol, polyethylene oxide, polyvinyl pyrrolidone, poly(ethylene imine), hyaluronic acid, gelatin, collagen, sodium alginate and hyaluronic acid, or a mixture and/or copolymer of two or more thereof.

9. The method of fabricating the vascular stent of claim 1, wherein the solvents each comprise at least one of water, hexafluoroisopropanol, dichloromethane, trichloromethane, tetrahydrofuran, N,N-dimethylformamide, dimethyl sulfoxide, acetone, n-propyl acetate and n-butyl acetate.

10. The method of fabricating the vascular stent of claim 1, further comprising:
subjecting the vascular stent to a heat treatment,
the heat treatment comprising:
placing the vascular stent in a heating medium and heating, holding and cooling the heating medium.

11. The method of fabricating the vascular stent of claim 10, wherein the heating medium comprises one of air, ethanol, ethylene glycol and glycerol, or a mixture of two or more thereof.

12. A method of fabricating a vascular stent system, comprising:
selecting a vascular stent fabricated in accordance with a method of fabricating the vascular stent as defined in any one of claims 1 to 11;
selecting a balloon and disposing the vascular stent over the balloon; and
placing the vascular stent in a crimping medium and crimping the vascular stent so that the vascular stent is crimped onto the balloon.

13. The method of fabricating the vascular stent system of claim 12, wherein the crimping medium comprises one of air, ethanol, ethylene glycol and glycerol, or a mixture of two or more thereof.

14. A vascular stent, comprising a stent body comprising a first fiber layer and a second fiber layer, which are stacked one on the other,
the first fiber layer formed of interwoven coaxial fibers each comprising a core layer and a cladding layer, the core layer produced from a biodegradable, water-insoluble polymer, the cladding layer produced from a biodegradable, water-soluble polymer, the cladding layer cladding the core layer,
the second fiber layer formed of interwoven solid fibers produced from a biodegradable, water-insoluble polymer.

15. A vascular stent system comprising a vascular stent as defined in claim 14 and a balloon, the vascular stent disposed over and crimped onto the balloon.
